Europäisches Patentamt

European Patent Office

Office européen des brevets

(11) Publication number: **0 196 752 B1**

# (12) EUROPEAN PATENT SPECIFICATION

(45) Date of publication of patent specification: **26.06.91** (51) Int. Cl.⁵: **G01N 33/569**, G01N 33/543

(21) Application number: **86300953.6**

(22) Date of filing: **12.02.86**

(54) Detecion of human T-cell Leukemia virus type III.

(30) Priority: **26.02.85 US 705708**

(43) Date of publication of application:
**08.10.86 Bulletin 86/41**

(45) Publication of the grant of the patent:
**26.06.91 Bulletin 91/26**

(84) Designated Contracting States:
**AT BE CH DE FR GB IT LI LU NL SE**

(56) References cited:
**EP-A- 0 125 893**
**WO-A-85/04903**
**GB-A- 2 114 289**
**US-A- 4 235 960**
**US-A- 4 297 104**

**THE LANCET, no. 8389, 9th June 1984, pages 1253-1256, The Lancet Ltd.; F. BRUN-VEZINET et al.: "Detection of IgG antibodies to lymphadenopathy-associated virus in patients with aids of lymphadenopathy syndrome"**

**THE LANCET, 30th June 1984, pages 1438-1440, The Lancet Ltd.; B. SAFAI et al.: "Seroepidemiological studies of human T-lymphotropic retrovirus type III in acquired**

immunodeficiency syndrome"

(73) Proprietor: **THE UNITED STATES OF AMERICA as represented by the Secretary, United States Department of Commerce National Technical Information Service, Office of Government Inventions and Patents, 5285 Port Royal Road Springfield, Virginia 22161(US)**

(72) Inventor: **Saxinger, Carl W.**
**6814 Renita Lane**
**Bethesda, MD 20817(US)**
Inventor: **Gallo, Robert Charles**
**8513 Thornden Terrace**
**Bethesda, MD 20834(US)**

(74) Representative: **Jump, Timothy John Simon et al**
**F.J. Cleveland and Company 40-43 Chancery Lane**
**London WC2A 1JQ(GB)**

## Description

The present invention is an enzyme linked immunoassay for the detection and measurement of small quantities of HTLV-III (AIDS). The assay process involves incubating human test sera suspected of containing AIDS human virus antibodies (HTLV-III antibodies) with HTLV-III virus protein bound to an insoluble immunosorbent. The amount of human immunoglobulin bound to the HTLV-III proteins is determined by first reacting said human antibodies with antibody against human immunoglobulins followed by reaction with a complex consisting of purified human immunoglobulin (1g) conjugated to peroxidase. The human 1g specifically binds to the HTLV-III antigens. The presence and measurement of test sera is determined by the addition of a hydrogen peroxidase solution which reacts with the peroxidase enzyme to form a peroxidase color reaction.

In this invention it is noted that the imnunoglobulin bonded to peroxidase is human immunoglobulin.

Evidence suggests that the retrovirus(es) of the HTLV family is an etiological agent of acquired immune deficiency syndrome (AIDS) based on the following: (1) there is precedence for an animal retrovirus cause of immune deficiency (feline leukemia virus in cats); (2) retroviruses of the HTLV family are T-cell tropic; (3) they preferentially infect "helper" T-cells (OKT4$^+$); (4) they have cytopathic effects on various human and mammalian cells as demonstrated by their induction of cell syncytia formation; (5) they can alter some T-cell functions; (6) in some cases infection may result in selective T-cell killing; and (7) they are transmitted by intimate contact or through blood products. The presence of antibodies directed to cell membrane antigens of HTLV infected cells has been shown in sera of more than 40% of patients with AIDS [Essex et al., Science, 220:859 (1983)]. This antigen has since been defined as part of the envelope of HTLV.

The original detection and isolation of the various HTLV isolates were made possible by two earlier developments: the discovery of T-cell growth factor (TCGF), also called Interleukin 2 (I1-2), which enabled the routine selective growth of different subsets of normal and neoplastic mature T-cells [Ruscetti, et al., J. Immunol., 119:131 (1977); and Poiesz, et al., Proc. Nat. Acad. Sci. USA, 77:6134 (1980)] and the development of sensitive assays for detection of retroviruses based on reverse transcriptase assays. The methods of HTLV isolation and transmission involved a cocultivation procedure using permissive T-cells for the virus. The use of normal human T-cells in cocultivation experiments preferentially yielded HTLV of both subgroups with immortalizing (transforming) capability for some of the target T-cells.

Disclosure of these discoveries is found in Gallo, et al., US-A-4,520,113 (publ. 28.05.85) and Gallo, et al., WO 85/04897 (publ. 07.11.85). Parallel references are Popovic, et al., "Detection, Isolation, and Continuous Production of Cytopathic Retroviruses (HTLV-III) from Patients with AIDS and Pre-AIDS," Science, 224:497, May 4, 1984; Schupbach, et al., "Serological Analysis of a Subgroup of Human T-Lymphotropic Retroviruses (HTLV-III) Associated with AIDS," Science, 224:503, May 4, 1984; and Sarngadharan, et al., "Antibodies Reactive with Human T-Lymphotropic Retroviruses (HTLV-III) in the Serum of Patients with AIDS," Science, 224:506, May 4, 1984. Other methods of detecting, isolating, and purifying HTLV-III virus or HTLV-III antibodies are disclosed in Gallo, et al., WO 85/04903 (publ. 07.11.85).

"The Lancet" 30th June 1984, page 1438-1440 discloses a method for the determination of AIDS antigens by an indirect ELISA method in which bound AIDS antibodies are reacted with peroxidase-labelled goat anti-human immunoglobulin. The use of unlabelled anti-human immunoglobulin is not discussed.

US-A-4,235,960 relates to a competitive enzyme-linked immunoassay by an ELISA method using two antibodies, wherein a first antibody is linked to a labelled antibody of the same species via a second bridging antibody against the first antibody and the labelled antibody.

GB-A-2,114,289 relates to indirect methods for measuring bound antibodies. However this immunoassay is for class specific immunoglobulin antibodies, and is not directed to improved AIDS assays of high sensitivity.

## Summary of the Invention

The present invention is another step in the discoveries leading to a therapeutic reagent for acquired immune deficiency syndrome (AIDS). Furthermore, the present invention represents an additional refinement in AIDS detection techniques.

In a first aspect of the invention there is provided a method for assaying to determine the amount of antibodies to human T-cell leukemia virus type III present, characterised in that:

(a) AIDS virus antibodies present in the sample are bonded to HTLV-III antigens linked to an insoluble immunosorbent and are incubated with anti-human immunoglobulin;

(b) peroxidase-labelled human Ig is added;

(c) a colour former is added which reacts with the peroxidase; and

(d) the amount of HTLV-III antibodies is indirectly measured by measuring activity of said peroxidase.

In a second aspect of the invention there is provided a method for assaying to determine the amount of antibodies to human T-cell leukemia virus type III present in a sample solution characterised in that

(a) HTLV-III- antibodies present in the sample are linked to an insoluble solid immunosorbent

(b) anti-human immunoglobulin is reacted with said antibodies;

(c) said anti-human immunoglobulin which is bound to said antibodies is further reacted with peroxidase-labelled human immunoglobulin, said human immunoglobulin specifically binding to said anti-human immunoglobulin;

(d) colour former is added which specifically reacts with said peroxidase; and

(e) activity of the peroxidase is measured in order to indirectly measure the amount of HTLV-III antibodies present in said sample solution.

In the process of the present invention it is of interest that the peroxidase enzyme is utilized in amounts which are termed greater enzymatic amounts and a preferred amount is to use a moderate excess of peroxidase which can be added to the immune material.

The Ig component of the process is selected from any available Ig antibody such as IgA, IgD, IgE, IgG, and IgM. A preferred temperature for the prccess is in the cold and specifically at or about 1°C. A range of 1-50°C may be tolerated. A preferred peroxidase is horseradish peroxidase. A preferred ratio of peroxidase to Ig or gamma globulin is 5:1.

Material Information Disclosure

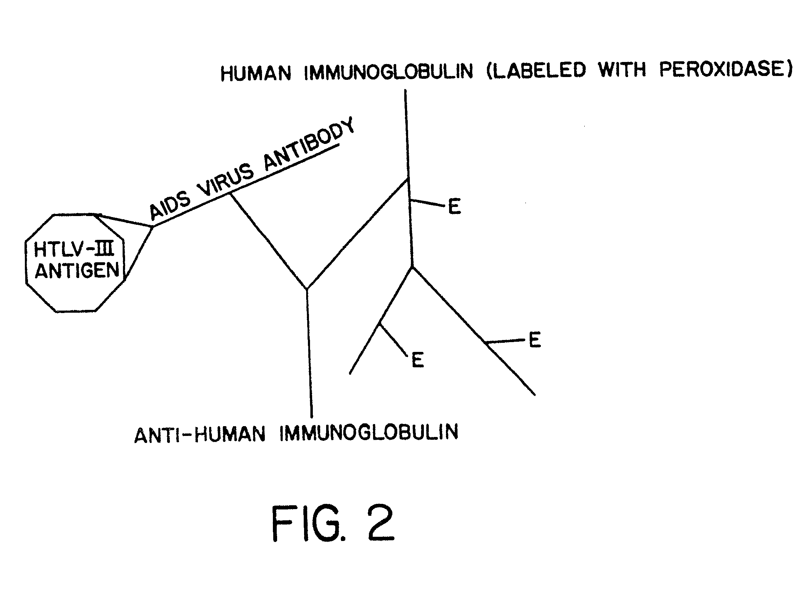

Sternberger, Immunocytochemistry, "The unlabelled Antibody Enzyme Method," Ludwig Prentice Hall, 1974, pp. 129-171, discloses an unlabelled antibody of PAP (peroxidase-antiperoxidase) procedure. The present invention is a modification of the Sternberger procedure, which is suitable for use with animals and uses peroxidase bound to antiperoxidase. This procedure only applies to animal reagents because a human PAP reagent is unavailable since it requires immunizing a human with peroxidase. Furthermore, the human PAP step of Sternberger is substituted in the present invention for human IgG labelled with peroxidase. See particularly Figure 2.

Maggio (ed.) Enzyme-Immunoassay , CRC Press, Inc., P. 44 et seq. (1980) and Nakane, et al., J. Histochem., Cytochem., Vol. 22, p. 1084 (1974) disclose methods of periodate coupling (peroxidase-labelled immunoglobulins) but do not disclose a method for detection of antibodies to HTLV-III.

Kurstak (ed.), Viral Immunodiagnosis, Academic Press, Inc., pp. 3-30 (1974) discloses a variety of immunoperoxidase techniques and represents the state of the art.

Sasse, et al., U.S. Patent No. 4,235,960, discloses a competitive, double-antibody enzyme-linked immunoassay.

Specific Disclosure

In the HTLV-III assay method of the present invention, anti-human immunoglobulin, which specifically binds to HTLV-III antibodies, is added to human test sera suspected of containing the AIDS virus antibodies previously coupled to solid immunosorbent test strips. The amount of immunoglobulin bound to the test sera is measured by an enzyme-inmunoassay wherein human immunoglobulin labeled with peroxidase is incubated with the test strips. The amount of HTLV-III is measured by measuring the amount of inmunoglobulin/peroxidase complex bound to the anti-human inmunoglobulin previously bound to HTLV-III antigens. A preferred methodology illustrating the above procedure is disclosed in Example 1 and illustrated in Figure 2.

The first immunoglobulin, anti-human IgG, IgA, IgD, IgE, or IgM is preferably prepared from goat. Other animal anti-human immunoglobulin may be used, such as rabbit, hamster, chicken, rat, guinea pig, sheep, horse, or mouse. These immunoglobulins are commercially available.

The human immunoglobulin/peroxidase complex is the label by which HTLV-III antigens can be detected and measured. This complex may be formed, in the preferred embodiment, by chemically coupling purified human immunoglobulin and peroxidase. One method of preparation of this complex is described by Nakane, et al., J. Histochem. Cytochem., Vol. 22, p. 1084 (1974). Horseradish peroxidase, a colorless protein (apoenzyme) combined with a dark brown iron-porphyrin (and composed of 47.0% C, 13.2% N, 7.35% H, 0.43% S and 0.12% Fe) is available commercially in a highly purified crystalline form. In the first step, reactive amino groups on HRP are blocked by alkylation with dinitrofluorobenzene (DNFB). Blocked HRP is then activated for coupling by periodate cleavage by the sequential addition of $NaIO_4$ and $H_2O$ (pH 8) followed by addition of immunoglobulin-$NH_2$ (pH 8) followed by addition of immunoglobulin-$NH_2$ and $NaBH_4$ (borohydride). The result is the IgG-peroxidase complex. Even though this coupling procedure is well known and commonly used, this invention may use other coupling methods. Although IgG is preferred, IgA, IgD, IgE, or IgM may be used.

The immunosorbent test strips used in the present invention are insoluble and capable of attaching HTLV-III antigens. This immunoadsorbent is present as an insoluble surface and may be agarose in bead form, carbohydrates such as dextran, cellulose, or nitrocellulose, plastics such as polystyrene, polycarbonate, polypropylene, or polyamide, or inorganic materials such as glass or silica gel. HTLV-III antigens may be chemically coupled to the immunosorbent or coated onto the surface of the immunosorbent.

The present invention employs the immunoglobulin specific combining property for two different purposes. The first purpose is the same as in labeled antibody methods; that is, immunoglobulin is used directly or indirectly as a reagent for selective localization of the tissue antigen under investigation. However, instead of visualizing this reaction by labeling the immunoglobulin, visualization is done by using specific immunoglobulin binding a second time, this time to bind onto the immunoglobulin an enzyme that is detectable by a suitable histochemical substrate. The double use of immunoglobulin specifically in sequence depends on the bivalence of the immunoglobulin.

The color former may be any chromogenic reagent which reacts with the peroxidase labeled human immunoglobulin in order to produce color. The preferred reagent contains hydrogen peroxide and dianisidine. Other chromogenic reagents conventionally used are o-phenylene diamine, p-phenylenediamine, 5-amino-salicylic acid, pyrogallol and similar solutions, such as diaminobenzidine.

The method for assaying HTLV-III antibodies may be incorporated into a test kit characterized by:

a) an insoluble immunosorbent which may be in bead, strip, plate, or test cavity form;

b) anti-human immunoglobulin capable of binding to HTLV-III antibodies;

c) human immunoglobulin labeled with peroxidase, said human immunoglobulin being capable of bonding to the anti-human immunoglobulin noted above;

d) a color former or chromogenic reagent suitable for reaction with peroxidase; and

e) a means for measuring the activity of the peroxidase after contact with the chromogenic reagent.

Description of the Figures

Figure 1 shows identification of HTLV-III in different test sera taken from suspected AIDS patients.

Figure 2 is a schematic diagram illustrating the invention.

Example 1

Identification of HTLV-III antigens recognized by Ugandan sera collected in 1972-1973.

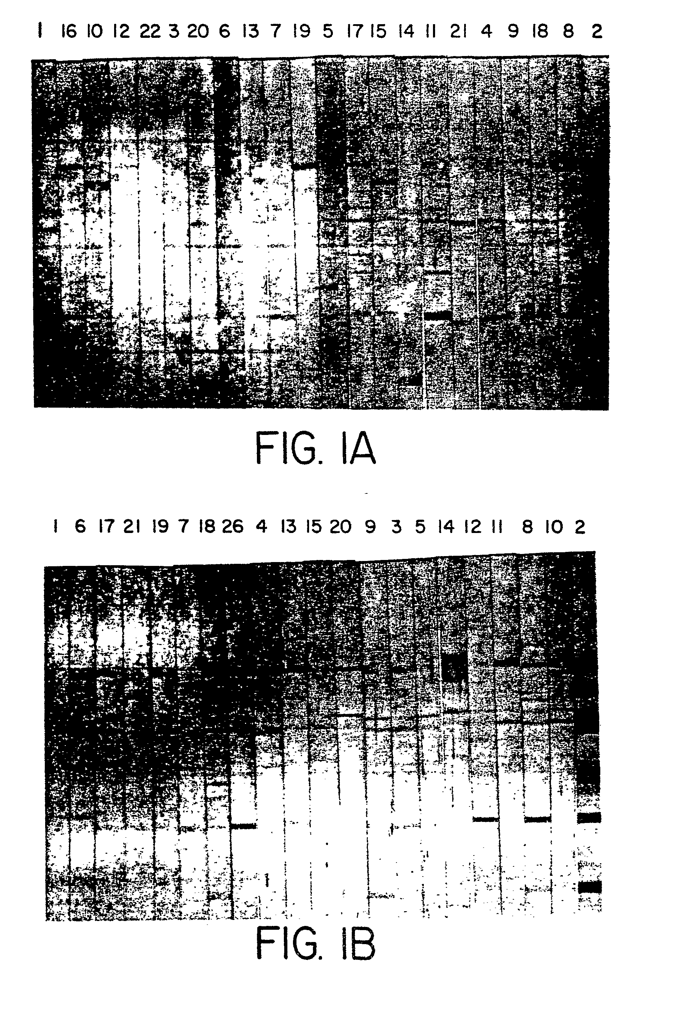

Absorbance values of samples tested for HTLV-III antibody were expressed in ratio to a standard normal control serum and samples with ratio values greater than the [(mean + 2sd) = 2.0] for normal donors were subjected to a confirmatory test requiring positive reactivity with the major HTLV-III viral bands separated by SDS-PAGE (200 μg of virus per slab, 12% acrylamide) and transferred to nitrocellulose sheets by electroblotting overnight at 15°C and 30V. The sheets were cut into strips and incubated with human test sera diluted 1:400 in 5% non-fat dried milk containing 0.02% merthiolate for 16 hours at 4°C. The strips were washed with wash Buffer, PBS containing 0.5% deoxycholate, 0.5% Triton® X-100 and 1 mM phenyl-, methyl-, sulfonylfluoride (PMSF). Next the strips were incubated at room temperature for 1 hour with goat antihuman IgG Fc fragment (Cappel labs) diluted 1:400 in Buffer 1, 20 mM Tris-HCl pH 7.5 containing 1mM EDTA, 0.2 M NaCl, 0.3% Triton® X-100, 2mg/ml bovine serum albumin, and 4% normal goat serum. The strips were washed as above and incubated for 1 hour at room temperature with human IgG conjugated with horseradish peroxidase (2 μg/ml) in PBS containing 0.5% Tween® 20, and 5% normal goat serum. After washing, the peroxidase color reaction was developed by incubation in 25 mM tris pH 7.5 containing 0.02% hydrogen peroxide and 0.025% o-dianisidine. Panels (A) and (B) of Figure 1 show 39 different sera tested in consecutive experiments. Strips are arranged in order of ascending screening ratios from left to right; lane numbers refer to the original position of the strip within the nitrocellulose blot. Lanes A1 and B1 contain normal control human serum; A2 and B2 contain positive control human serum from an AIDS patient.

**Claims**

1. An assay method for the determination of the amount of antibodies to human T-cell leukemia virus type III present, characterised in that:

(a) AIDS virus antibodies present in the sample are bonded to HTLV-III antigens linked to an insoluble immunosorbent and are incubated with anti-human immunoglobulin;

(b) peroxidase-labelled human Ig is added;

(c) a colour former is added which reacts with the peroxidase; and

(d) the amount of HTLV-III antibodies are indirectly measured by measuring activity of said peroxidase.

2. An assay method for the determination of the amount of antibodies to human T-cell leukemia virus type III present in a sample solution characterised in that

(a) HTLV-III- antibodies present in the sample are linked to an insoluble solid immunosorbent

(b) anti-human immunoglobulin; are reacted with said antibodies;

(c) said anti-human immunoglobulin which are bound to said antibodies are further reacted with peroxidase-labelled human immunoglobulin, said human immunoglobulin specifically binding to said anti-human immunoglobulin;

(d) colour former is added which specifically reacts with said peroxidase; and

(e) activity of the peroxidase is measured in order to indirectly measure the amount of HTLV-III antibodies present in said sample solution.

3. The method of claim 2, characterised in that antihuman immunoglobulin is one member selected from the group of immunoglobulins consisting of IgA, IgD, IgE, IgG and IgM.

4. The method of claim 3, characterised in that said immunoglobulin is animal immunoglobulin.

5. The method of claim 2, characterised in that said insoluble solid immunosorbent is selected from one member of the group consisting of agarose in bead form, carbohydrates such as dextran, cellulose, or nitrocellulose, plastics such as polystyrene, polycarbonate, polypropylene, or polyamide, and inorganic material such as glass or silica gel.

6. A method according to any of claims 2 to 5 characterised in that

a) a sample solution suspected of containing HTLV-III antibodies or antibodies exhibiting known HTLV-III specificity is contacted with an insoluble immunosorbent under conditions in which said antibodies will link to said immunosorbent;

b) anti-human immunoglobulin are added to said antibodies linked to said immunosorbent under conditions in which said immunoglobulin will bond to said antibodies; and

c) peroxidase-labelled human immunoglobulin is added to said immunosorbent under conditions in which said human immunoglobulin will bond to said anti-human immunoglobulin;

7. A test kit for assaying HTLV-III antibodies characterised by

a) an insoluble immunosorbent in bead, strip, plate, or test cavity form;

b) anti-human immunoglobulin capable of bonding to HTLV-III antibodies;

c) human immunoglobulin labelled with peroxidase, said human immunoglobulin being capable of bonding to the anti-human immunoglobulin noted above;

d) a colour former or chromogenic reagent suitable for reaction with peroxidase; and

e) a means for measuring the activity of the peroxidase after contact with the chromogenic reagent.

**Revendications**

1. Méthode de dosage pour la détermination de la quantité présente d'anticorps dirigés contre le virus type III de la leucémie des cellules T humaines, caractérisée en ce que :

a) les anticorps dirigés contre le virus du SIDA, présents dans l'échantillon, sont liés aux antigènes HTLV-III liés à un immunosorbant insoluble et sont incubés avec de l'immunoglobuline anti-humaine ;

b) de l'Ig humaine marquée à la peroxydase est ajoutée ;

c) un formateur de couleur, réagissant avec la peroxydase, est ajouté ; et

d) la quantité d'anticorps anti-HTLV-III est mesurée directement par mesure de l'activité de ladite peroxydase.

2. Méthode de dosage pour la détermination de la quantité, présente dans une solution d'échantillon, d'anticorps dirigés contre le virus type III de la leucémie des cellules T humaines, caractérisée en ce que :

a) les anticorps anti-HTLV-III, présents dans l'échantillon, sont liés à un immunosorbant solide insoluble ;

b) de l'immunoglobuline anti-humaine est mise à réagir avec lesdits anticorps ;

c) ladite immunoglobuline anti-humaine, qui est liée auxdits anticorps, est mise ensuite à réagir avec l'immunoglobuline humaine marquée à la peroxydase, ladite immunoglobuline humaine se liant spécifiquement à ladite immunoglobuline anti-humaine ;

d) un formateur de couleur, réagissant spécifiquement avec ladite peroxydase, est ajouté ; et

e) l'activité de la peroxydase est mesurée afin de déterminer indirectement la quantité d'anticorps anti-HTLV-III présente dans ladite solution d'échantillon.

3. Méthode selon la revendication 2, caractérisée en ce que l'immunoglobuline anti-humaine est une des immunoglobulines choisie parmi l'IgA, l'IgD, l'IgE, l'IgG et l'IgM.

4. Méthode selon la revendication 3, caractérisée en ce que ladite immunoglobuline est de l'immunoglobuline d'origine animale.

5. Méthode selon la revendication 2, caractérisée en ce que ledit immunosorbant solide insoluble est choisi dans le groupe constitué par l'agarose sous forme de perles, les hydrates de carbone tels que le dextrane, la cellulose ou la nitrocellulose, les matières plastiques telles que le polystyrène, le polycarbonate, le polypropylène ou le polyamide, et les substances minérales telles que le verre ou le gel de silice.

6. Méthode selon l'une quelconque des revendications 2 à 5, caractérisée en ce que
   a) une solution d'échantillon, suspectée de contenir des anticorps anti-HTLV-III ou des anticorps présentant une spécificité connue anti-HTLV-III, est mise en contact avec un immunosorbant insoluble dans les conditions où lesdits anticorps se lient audit immunosorbant ;
   b) de l'immunoglobuline anti-humaine est ajoutée auxdits anticorps liés audit immunosorbant, dans les conditions dans lesquelles ladite immunoglobuline se lie auxdits anticorps ; et
   c) de l'immunoglobuline humaine, marquée à la peroxydase, est ajoutée audit immunosorbant, dans les conditions où ladite immunoglobuline humaine se lie à ladite immunoglobuline anti-humaine.

7. Trousse diagnostique pour le dosage d'anticorps anti-HTLV-III, caractérisé par
   a) un immunosorbant insoluble sous forme de perles, de bande, de plaque ou de cavité de dosage;
   b) de l'immunoglobuline anti-humaine capable de se lier aux anticorps anti-HTLV-III ;
   c) de l'immunoglobuline humaine, marquée à la peroxydase, ladite immunoglobuline humaine étant capable de se lier à l'immunoglobuline anti-humaine mentionnée ci-dessus ;
   d) un formateur de couleur ou un réactif chromogène approprié pour la réaction avec la peroxydase ; et
   e) un moyen pour mesurer l'activité de la peroxydase après la mise en contact avec le réactif chromogène.

**Ansprüche**

1. Untersuchungsverfahren zur Bestimmung der vorhandenen Menge von Antikörpern zum menschlichen T-Zellen-Leukämievirus Typ III, dadurch gekennzeichnet, daß:
   a) in der Probe vorhandene AIDS-Virus-Antikörper an HTLV-III-Antigene gebunden werden, die an ein unlösliches Immunsorptionsmittel gekoppelt sind, und mit Antihuman-Immunglobulin inkubiert werden,
   b) Peroxidase-markiertes menschliches Ig zugegeben wird,
   c) ein Farbbildner zugegeben wird, der mit der Peroxidase reagiert, und
   d) die Menge von HTLV-III-Antikörpern durch Messung der Aktivität der Peroxidase indirekt gemessen wird.

2. Untersuchungsverfahren zur Bestimmung der Menge von in einer Probenlösung vorhandenen Antikörpern zum menschlichen T-Zellen-Leukämievirus Typ III, dadurch gekennzeichnet, daß:
   a) in der Probe vorhandene HTLV-III-Antikörper an ein unlösliches festes Immunsorptionsmittel gekoppelt werden,
   b) Antihuman-Immunglobulin mit den Antikörpern zur Reaktion gebracht wird,
   c) das an die Antikörper gebundene Antihuman-Immunglobulin mit Peroxidase-markiertem Human-Immunglobulin weiter reagiert wird, wobei sich das Human-Immunglobulin spezifisch an das Antihuman-Immunglobulin bindet,
   d) ein Farbbildner zugegeben wird, der spezifisch mit der Peroxidase reagiert, und
   e) die Aktivität der Peroxidase als indirektes Maß der in der Probenlösung vorhandenen Menge von HTLV-III-Antikörpern gemessen wird.

3. Verfahren nach Anspruch 2, dadurch gekennzeichnet, daß das Antihuman-Immunglobulin aus der Gruppe der IgA, IgD, IgE, IgG und IgM umfassenden Immunglobuline ausgewählt wird.

4. Verfahren nach Anspruch 3, dadurch gekennzeichnet, daß das Immunglobulin tierisches Immunglobulin ist.

5. Verfahren nach Anspruch 2, dadurch gekennzeichnet, daß das unlösliche feste Immunsorptionsmittel aus einer Gruppe ausgewählt wird, die Agarose in gekörnter Form, Kohlenhydrate wie beispielsweise Dextran, Zellulose oder Nitrozellulose, Kunststoffe wie beispielsweise Polystyrol, Polycarbonat, Polypropylen oder Polyamid, und anorganisches Material wie beispielsweise Glas oder Silicagel umfaßt.

6. Verfahren nach einem der Ansprüche 1 bis 5, dadurch gekennzeichnet, daß:

   a) eine mutmaßlich HTLV-III-Antikörper oder Antikörper, die bekanntes HTLV-III-spezifisches Verhalten zeigen, enthaltende Probenlösung mit einem unlöslichen Immunsorptionsmittel unter Bedingungen in Kontakt gebracht wird, unter denen die Antikörper sich an das Immunsorptionsmittel koppeln,

   b) Antihuman-Immunglobulin zu den an das Immunsorptionsmittel gekoppelten Antikörpern unter Bedingungen zugegeben wird, unter denen das Immunglobulin sich an die Antikörper bindet, und

   c) Peroxidase-markiertes Human-Immunglobulin zu dem Immunsorptionsmittel unter Bedingungen zugegeben wird, unter denen das Human-Immunglobulin sich an das Antihuman-Immunglobulin bindet.

7. Testausrüstung zur Untersuchung von HTLV-III-Antikörpern, gekennzeichnet durch

   a) ein unlösliches Immunsorptionsmittel in gekörnter, Streifen-, Platten-, oder Testhohlraumform,

   b) Antihuman-Immunglobulin, das an HTLV-III-Antikörper bindefähig ist,

   c) Human-Immunglobulin, das mit Peroxidase markiert ist und an das obengenannte Antihuman-Immunglobulin bindefähig ist,

   d) ein Farbbildner oder chromogenes Reagens, das sich für eine Reaktion mit Peroxidase eignet, und

   e) Mittel zur Messung der Aktivität der Peroxidase nach Kontakt mit dem chromogenen Reagens.

I 16 10 12 22 3 20 6 13 7 19 5 17 15 14 11 21 4 9 18 8 2

# FIG. IA

I 6 17 21 19 7 18 26 4 13 15 20 9 3 5 14 12 11 8 10 2

# FIG. IB

HUMAN IMMUNOGLOBULIN (LABELED WITH PEROXIDASE)

AIDS VIRUS ANTIBODY

HTLV-III ANTIGEN

E

E

E

ANTI-HUMAN IMMUNOGLOBULIN

FIG. 2

EP 0 196 752 B1